(19) Europäisches Patentamt
European Patent Office
Office européen des brevets

(11) Publication number: **0 054 987**
**B1**

(12) **EUROPEAN PATENT SPECIFICATION**

(45) Date of publication of patent specification: **25.01.89**

(21) Application number: **81201290.4**

(22) Date of filing: **20.11.81**

(51) Int. Cl.⁴: **C 12 P 1/00,** C 12 N 11/00, C 12 P 7/02 // C12P7/04, C12P7/62, C12P17/02

(54) **Process for microbial transformation using immobilised cells, and immobilised cell systems.**

(30) Priority: **12.12.80 GB 8039986**

(43) Date of publication of application:
**30.06.82 Bulletin 82/26**

(45) Publication of the grant of the patent:
**25.01.89 Bulletin 89/04**

(84) Designated Contracting States:
**BE CH DE FR GB IT LI NL**

(56) References cited:
**EP-A-0 007 176**
**EP-A-0 035 883**
**FR-A-2 019 322**
**FR-A-2 340 979**
**GB-A-2 004 300**
**GB-A-2 018 772**
**GB-A-2 035 359**

**RUSSIAN CHEMICAL REVIEWS, Uspekhi Khimii, vol. 49, May 1980, no. 5, LONDON (GB), K. MARTINEK and I.B. BEREZIN: "The stabilisation of enzymes- a key factor in the practical application of biocatalysis", pp. 385-403**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Bolsman, Theodorus Antonius Benedictus Maria**
**Badhuisweg 3**
**NL-1031 CM Amsterdam (NL)**
Inventor: **Bailey, Maureen Linda**
**53 Scott Close, Ditton**
**Nr. Maidstone, Kent (GB)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention relates to the microbial transformation of organic substrates, and in particular to the use of cells in an immobilised system to effect the transformation of a substantially water-immiscible organic substrate.

Micro-organisms are known to be able to effect a variety of commercially useful chemical transformations in a highly selective manner at, or near, ambient temperature and pressure. However, the majority of the industrially attractive feedstocks are highly apolar and hence substantially immiscible with water, whilst the micro-organisms themselves require an aqueous environment and cannot survive in an organic apolar medium. Hitherto, this need for two immiscible phases has been resolved by the use of an emulsion containing the desired organic substrate as one phase and a second aqueous phase containing the appropriate micro-organism. However, such two-phase emulsion systems introduce a number of practical problems including the risk of physical damage to the microbial cells resulting from the shear forces required to maintain the emulsion, and of chemical damage from any surfactants which may be used to stabilise the emulsion with minimal physical force. Furthermore, the mixing in such an emulsion exposes the cells to the deleterious effects of direct contact with the organic phase, and at the same time increases the practical problems involved in separating the desired organic product from the intimate mixture of organic and aqueous cell-containing phases in the emulsion. It has now been found that these problems can be circumvented or mitigated by the use of an immobilised microbial system in which the micro-organism is located in the water-filled pores of a porous particulate support.

In this immobilised system, the micro-organism can be introduced into the potentially hostile medium of a water-immiscible organic liquid, whilst it continues to enjoy the aqueous micro-environment formed by the water held in the pores of the support material. Thus, the micro-organism is sheltered from the deleterious consequences of direct contact with the apolar organic liquid. However, no two liquids are wholly immiscible and the organic phase therefore transfers to a limited extent into the aqueous phase. There it is exposed to the biochemical action of the micro-organism, and the organic products thus formed will selectively transfer back into the organic phase in consequence of their greater affinity for that phase, i.e. their partition coefficients will favour the organic phase. Thus, a further potential benefit of this immobilised system is that reaction products are extracted from the microbial environment into the organic phase before they can assume a concentration high enough to endanger the continued biochemical activity of the microorganism, and also before they experience a further transformation to other, less desired products.

GB Patent Application No. 2018772 describes the microbial oxidation of alkanes to ketones or secondary alcohols and of secondary alcohols to ketones. The specification teaches that the oxidation should be carried out using air as the oxygen source.

The present invention comprises a process for the microbial oxidation of a substantially water-immiscible organic substrate wherein a micro-organism species capable of effecting the required oxidation of said substrate is located on a porous, water-bearing, particulate inert support, the pore size being greater than the mean diameter of the micro-organism, thereby forming an immobilised system in which the micro-organism has an aqueous environment, and the immobilised system is contacted with the substrate and a source of oxygen, the source of oxygen being a gaseous mixture containing more than 21% but not more than 75% by vol. of oxygen. Many micro-organisms are known to have the capability of oxidising various organic substrates and in principle any such micro-organism may be used in the process of this invention. In practice the choice of micro-organism will naturally be dictated by the nature of the substrate and the transformation which it is desired to effect. One useful application of the present process is in the oxidation of an aliphatic hydrocarbon, and a micro-organism suitable for such oxidations is the known species *Pseudomonas aeruginosa* 473, which is capable of oxidising an alkane to the corresponding 1-alkanol and a 1-alkene to the corresponding 1,2-epoxy alkane.

This organism was first described by Thijsse and van der Linden in 1958 (Ant. v. Leeuwenhoek *24*, 298 (1958), and has the following morphological and biochemical characteristics:—

| | |
|---|---|
| Cell morphology | Medium rods<br>Off-white with a greenish tinge.<br>Flat, smooth, entire with a slight margin, circular, translucent. |
| °C growth | 45°C+<br>50°C− |
| Catalyse | + |
| Oxidase, Kovacs | + |
| O—F glucose | oxidative |
| Gram | negative |
| Pyocyanin | + |
| Fluorescence | + |
| Penicillin G | − |
| Streptomycin | + |
| Chlorophenicol | + |

| | |
|---|---|
| Tetracycline | + |
| Novobiocin | − |
| Polymyxin B | + |
| 0/129 | − |
| Levan | − |

The support material should naturally be chemically and biologically inert under the conditions of use, and suitable materials are inorganic oxides such as silica, alumina, zirconia, magnesia and mixtures thereof. The achievement of an effective activity in the immobilised microbial cells is dependent on the size of the pores in the support particles being above a critical minimum, and as indicated above this should be greater than the mean diameter of the micro-organism, which normally requires a pore size greater than 0.5 micron. The size of the support particles often influences the activity of the immobilised system, larger particles yielding a higher activity. The precise combination of pore size and particle size required to achieve maximum microbial biochemical activity will naturally depend on the micro-organism involved, the detailed chemistry and morphology of the support material, and the nature of the substrate; in the case of *Pseudomonas aeruginosa* supported on alumina, and used in the oxidation of methylcyclohexane, it has been found that the highest levels of activity and longevity are attained when the particle size is at least 1 mm, and especially at least 6 mm.

It has also been found that in many cases the activity and longevity of the cells located on the support can be enhanced if the support surface has a lipophilic coating. In the case of silica supports, such a coating may be applied by acid treatment followed by silylation of the hydroxyl groups thereby exposed. Alternatively the support may be impregnated with a polymer such as polystyrene.

The location of the cells onto the support may be carried out by a variety of techniques, but two procedures have been found to be generally suitable. In one technique the microbial cells are cultivated by established methods in a fermenter so as to yield a paste of the required cells. The support particles are subjected to a reduced pressure, the cell paste applied to the support whilst maintaining the reduced pressure, and atmospheric pressure then restored so as to force the paste into the pores. An alternative technique is that of growth in situ, in which the particulate carrier is suspended in a suitable growth medium, which is then inoculated with the desired organism. The inoculated, support-containing growth medium is then incubated under appropriate conditions to provide for growth of the organism, and after a suitable period, free cells are removed by washing to leave the cell-containing immobilised system. In a variation of this technique, fresh growth medium containing an assimilable source of carbon and nitrogen may be continuously fed into the reactor during immobilisation to provide suitable conditions for attachment.

The yield of the desired product can often be enhanced by the addition to the organic phase of a compound which reduces the ability of the micro-organism to progress the transformation beyond the desired stage. Thus, oxidation of a hydrocarbon to an alkanol may be improved by the addition to the organic phase of a different alcohol; without being limited by any particular mechanism it is believed that the alcohol serves to saturate the dehydrogenase enzyme site in the micro-organism and thereby minimise further oxidation of the desired product alcohol. Suitable alcohols for this inhibitory function include nonanol and methanol, though in the latter case the concentration should not be so high as to lead to inhibition of the micro-organism.

It has also been found that oxidation of the substrate under conditions of oxygen limitation (which will often apply) is influenced by the concentration of oxygen in the ambient atmosphere to which the culture system is exposed, and that although an increase in oxygen partial pressure produces an increased conversion of substrate to oxidised product, there is a limit to this effect beyond which increased oxygen concentration becomes inhibitory. Preferably the concentration of oxygen should exceed the 21% present in the atmosphere but not be greater than 75%.

The invention is illustrated in the following Examples.

## Example I

*Pseudomonas aeruginosa* 473 was grown in a Biotec fermentor using either batch culture in ASM medium with heptane vapour as carbon source, or continuous culture in AM—2 medium with 0.33 g/l polypropylene glycol 2000 as antifoam, heptane vapour as carbon source and a stirring speed of 600 rpm. This organism was then immobilised on a particulate support by further growth in the presence of the support using the following procedure.

The composition of the media were as follows:—

*Composition of medium ASM*

| | $g\ l^{-1}$ |
|---|---|
| $NH_4Cl$ | 0.535 |
| $KH_2PO_4$ | 0.531 |
| $Na_1HPO_4$ | 0.866 |
| $K_2SO_4$ | 0.174 |
| $MgSO_4\ 7H_2O$ | 0.037 |
| $CaCl_2\ 2H_2O$ | 0.00735 |
| $FeSO_4\ 7H_2O$ | 0.006g |

together with 1 ml per litre of the following mineral salts solution:—

| | | |
|---|---|---|
| $ZnSO_4\ 7H_2O$ | 1.44 | gram/5 l |
| $MnSO_4\ 4H_2O$ | 1.12 | |
| $H_3BO_3$ | 0.309 | |
| $CuSO_4\ 5H_2O$ | 0.624 | |
| $Na_2MoO_4\ 2H_2O$ | 0.242 | |
| $CoCl_2\ 6H_2O$ | 0.238 | |
| KI | 0.415 | |
| IM $H_2SO_4$ | 5 ml | |

*Composition of medium AM 2*

| | $g\ l^{-1}$ |
|---|---|
| $(NH_4)_2SO_4$ | 1.45 |
| $H_3PO_3$ (90%) | 1.09 |
| $MgSO_4\ 7H_2O$ | 0.099 |
| $CaCl_2\ 2H_2O$ | 0.015 |
| $FeSO_4\ 7H_2O$ | 0.02 |

together with 2 ml per litre of the mineral salts solution defined above.

50 ml of ASM medium containing 20 g of the selected support particles in a centre-well flask were inoculated with P.a. and incubated for 3 days at 30°C in a Grant shaker at 100 strokes per minute. After this growth period the support was washed with 0.1.M phosphate buffer (pH 6.8), the support particles removed and surplus, external water removed by drying on tissue paper.

The cell loading present in the resultant immobilised cell system was determined by heating 1 g. of cell-loaded particles to 80°C in 5 ml of 2% sodium carbonate in 0.1 N aqueous sodium hydroxide for 2½ hours, followed by protein analysis according to the procedure of Folin-Ciocalteu (Methods in Enzymology Ed. Colowick & Kaplan, Vol. III, p. 447 Acad. Press Inc. N.Y. (1957)).

10 g of the immobilised cell system thus obtained were added to 15 ml of methylcyclohexane saturated with water in a 250 ml stoppered conical flask. This was agitated in an orbital shaker at 105 rpm and 30°C, 0.1 ml samples taken at defined intervals and, after addition of 0.5 ml of a 0.3 g/l solution of 1-(1-hydroxy)ethylcyclohexane in isooctane as internal reference, analysed by g.l.c. using a "Chromosorb" WHP glass column of 6 ft at 75°C with nitrogen as carrier gas at 30 ml/min and a flame ionisation detector.

The results obtained with a selection of support materials are shown in Table 1 below.

TABLE 1

| Support Material | SA 5202 | SA 5205 | | SA 5221 | | SA 5239 | | SS 5231 |
|---|---|---|---|---|---|---|---|---|
| Chemical Composition | 99.6% $Al_2O_3$ | 86% $Al_2O_3$ | | 89% $Al_2O_3$ | | 86% $Al_2O_3$ | | 95% $SiO_2$ |
| Pore Size (micron) | 0.5 - 4 | 50 - 300 | | 10 - 30 | | 1-2/50 - 200 | | 1-2/15 - 100 |
| Apparent Porosity (%) | 54 - 60 | 49 - 55 | | 47 - 52 | | 53 - 65 | | 34 - 38 |
| Surface area ($m^2/g$) | 0.7 - 1.3 | 0.005 - 0.05 | | 0.02 - 0.07 | | 0.15 - 0.45 | | 0.1 - 0.4 |
| Attrition resistance (%) | 68% | 0% | | 3% | | 2 | | 8 |
| Immobilisation time (days) | 6 | 3 | 6 | 3 | 6 | 3 | 6 | 6 |
| Cell loading ($x10^{-3}$) | 0.4 | 0.4 | 0.8 | 0.54 | 1 | 0.6 | 1 | 0.13 |
| Formation of product ($x10^{-3}$) | 4 | 7 | 1 | 3.4 | 1.8 | 3.0 | 1.0 | 2.0 |
| Biocatalyst lifetime (h) | 110 | 80 | 120 | 100 | 120 | 50 | 90 | 20 |

All support materials were obtained from the Norton Company and are identified by the reference number allocated by Norton.

Attrition resistance is determined as the percentage weight loss after shaking for 6 days in Grant shaker at 180 strokes per minute in water.

Cell loading is expressed as grams dry cells per gram support.

Product formation is expressed as gram methylcyclohexanol formed per gram dry cells per hour, integrated over a period of 50 hours.

Example II

The influence of the particle size of the support material was evaluated by selecting closely defined ranges of particle size and assessing the cell loading and activity attained using procedures as described in Example I. The results obtained are set out in Table 2 below.

TABLE 2

| Support | Particle size (mm) | Loading (g dry cells g support $^{-1}$) | Activity (g products $h^{-1}$ g dry cells $^{-1}$) | Life time (h) |
|---|---|---|---|---|
| SA 5221 | 6-7 | $1.2 \times 10^{-3}$ | $6 \times 10^{-3}$ | > 140 |
| SA 5221 | 1-2 | $0.65 \times 10^{-3}$ | $2.2 \times 10^{-3}$ | 100 |
| SA 5221 | 0.6-1 | $1.5 \times 10^{-3}$ | $4.2 \times 10^{-3}$ | > 140 |
| SA 5221 | 0.3-0.6 | $1.1 \times 10^{-3}$ | $3.6 \times 10^{-3}$ | 100 |
| SA 5205 | 7-8 | $0.28 \times 10^{-3}$ | $4 \times 10^{-3}$ | 80 |
| SA 5205 | 1-2 | $0.39 \times 10^{-3}$ | $2 \times 10^{-3}$ | 50 |
| SA 5205 | 0.6-1 | $0.5 \times 10^{-3}$ | $2.4 \times 10^{-3}$ | 100 |
| SA 5205 | 0.3-0.6 | $0.1 \times 10^{-3}$ | $10 \times 10^{-3}$ | 130 |

Example III

The influence of the procedure adopted for loading the support particles with the micro-organism cells was evaluated by comparing the growth in situ procedure described in Example I with the following alternatives. In all cases the support material was Norton alumina SA 5221 of particle size 6—7 mm, and the micro-organism was P.a.

a) Adsorption from concentrated cell suspension. 50 ml of 0.1M phosphate buffer (pH = 6.8) containing approx. 10 g of dry cells/l was shaken with 20 g of support beads for 2—3 days in a Grant shaker at 100 strokes per minute.

b) Vacuum immobilisation. 30—40 g of support beads were evacuated in a 300 ml septum-sealed Buchner flask to approx. 0.7 k Pa. 0.2 ml per gram of support of a concentrated suspension of micro-organism cells in 0.1 M phosphate buffer (pH 6.8) was then introduced with a syringe through the septum. After shaking the mixture thoroughly the vacuum was released suddenly.

In each case the loaded support particles were superficially dried with tissue paper, and assessed for their ability to oxidise methylcyclohexane to the corresponding alcohol as described in Example I.

The results are set out in Table 3 below.

TABLE 3

| Immobilisation Method | Growth in situ | Adsorption from concn. suspension | Vacuum Adsorption |
|---|---|---|---|
| Rate of product formation ($\mu$ mole/hr/g dry cells) | 4.4 | 17.5 | 13.2 |

Example IV

The applicability of the immobilised cell system in the transformation of different water-immiscible organic substrates was evaluated by replacing the methylcyclohexane of Example 1 with n-heptane, 1-octene, D,L-menthylacetate or with hexanol/hexanonic acid. In the case of menthyl acetate hydrolysis the micro organism used was *Bacillus subtilis* T372 (NCIB 11705); in the other cases it was *Pseudomonas aeruginosa* 473. FOr some of these systems comparative experiments were carried out using free cells as 2 ml of cell suspension in phosphate buffer (0.1M; ph 6.8) in 5 ml of the relevant organic phase. Some experiments were also carried out in which the organic substrate was used as a solution in isooctane. In those cases using an immobilised system the support material was Norton alumina SA 5221 loaded by vacuum immobilisation to give a cell loading of about $7 \times 10^{-3}$ g dry cells/g of support, except in the esterification experiments where the cell loading was $11.2 \times 10^{-3}$ g dry cells/g of support. The experimental conditions and procedures were otherwise as described in Ex. 1.

The results obtained are set out in Table 4 below.

TABLE 4

| Substrate(s)* | Product | Reaction System | Rate of Product formation (μ mole/hr/g. dry cells, integrated over first 50 hrs) | Catalyst lifetime (hr) |
|---|---|---|---|---|
| Heptane 100% | 1-Heptanol | Immobilised | 16 | > 200 |
| 1-Octene 100% | 1,2-epoxyoctane | " | 22 | 300 |
| " 20% | " | " | 29 | 300 |
| " 100% | " | Free | 0 | < 5 |
| " 20% | " | Free | 24 | 300 |
| D,L-menthyl acetate 100% | Menthol | Immobilised | 31 | >185 |
| " " | " | Free | 27 | 80 |
| " 10% | " | Immobilised | 13 | >185 |
| " 10% | " | Free | 20 | >185 |
| Hexanol 1 Hexanoic Acid 0.66 | n-hexyl-n-hexanoate | Free | 80 | 60** |
| 1 0.66 | " | Immobilised | 80 | 60** |
| 4 2.7 | " | Free | 310 | 50 |
| 4 2.7 | " | Immobilised | 200 | 70 |
| 7 4.7 | " | Free | 370 | 70 |
| 7 4.7 | " | Immobilised | 260 | 100 |
| 10 6.6 | " | Free | 440 | 70 |
| 10 6.6 | " | Immobilised | 340 | 100 |

* Substrate used either pure (100%), or in isooctane solution, when % = Vol % in isooctane
** Reaction complete. No substrate left.

Example V

In the oxidation of an aliphatic hydrocarbon to the corresponding alcohol, the influence on productivity of a different alcohol in the organic phase was determinal by two experiments. P.a. was vacuum immobilised onto Norton SA 5221 alumia (particle size 1—2 mm) following a procedure similar to that described in Ex. III(b), to give an immobilised system having a cell loading of 0.86 × $10^{-3}$ g dry cells/g support. This was then evaluated following the general procedure described in Example 1 and using, as substrate, heptane containing varying amounts of 1-nonanol. A similar experiment was also carried out using, as substrate, methylcyclohexane containing varying amounts of methanol. The results of these two experiments are shown in Tables 5 and 6 respectively.

TABLE 5

| Nonanol Conc. | Initial rate of Product Formation (g/hr/g dry cells)* | Lifetime (hr) |
|---|---|---|
| 0.1% | 0.4 x $10^{-3}$ | 50 |
| 0.5% | 1.4 x $10^{-3}$ | >150 |
| 1.0% | 3.7 x $10^{-3}$ | >150 |

* = integrated over the first 50 hours

TABLE 6

| Methanol Added (%) | Product formation (g/hr/g dry cells) | Lifetime (hr) |
|---|---|---|
| 0 | 3 x $10^{-3}$ | 100 |
| 0.05 | 5.8 x $10^{-3}$ | 90 |
| 0.1 | 5.8 x $10^{-3}$ | 90 |
| 0.2 | 4.0 x $10^{-3}$ | 90 |
| 0.23 | 2.6 x $10^{-3}$ | 60 |
| 0.5 | 1.9 x $10^{-3}$ | 20 |
| 1 | 1.05 x $10^{-3}$ | 20 |

Example VI

The influence of the ambient oxygen concentration on the productivity of an immobilised system was evaluated using P.a. vacuum immobilised onto Norton SA 5221 alumina (cell loading 3.2 × $10^{-3}$ g dry cells/g support). This system was assessed for ability to oxidise methylcyclohexane to the corresponding alcohol using a procedure similar to that described in Example 1 but employing differing oxygen levels; the results are set out in Table 7.

TABLE 7

| Oxygen Concn. (%) | Initial rate of Product Formation (g/hr/g dry cells)* | Lifetime (hr) |
|---|---|---|
| 21 | $1.8 \times 10^{-3}$ | 120 |
| 50 | $3.0 \times 10^{-3}$ | 120 |
| 75 | $4.6 \times 10^{-3}$ | 120 |
| 100 | $2.7 \times 10^{-3}$ | 120 |

* = integrated over the first 50 hours

Example VII

The effect of a lipophilic support coating was evaluated as follows

(a) *Acid treatment of silica*

100 g of Norton Silica (SS 5131) of bead size 7—8 mm was refluxed in 150 ml of 0.6 M sulphuric acid for 24 h. After increasing the acidity to 1.2 M the mixture was refluxed for another 3 days. After cooling, the beads were washed with tapwater for 48 hours and subsequently dried at 100°C for 17 h. This procedure resulted in a surface hydroxylated silica with approx. 3 hydroxyl groups/$nm^2$ (as determined by differential thermal analysis) and a surface area of 0.7 $m^2$ $g^{-1}$ (as determined by BET).

(b) *Silylation of rehydrated silica*

30 g of rehydrated silica was refluxed with $3.55 \times 10^{-2}$ mole of a compound with general composition

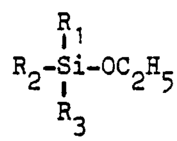

in 30 ml toluene for 19 hr. under a nitrogen atmosphere. Subsequently the support was washed with toluene in a Soxlet apparatus for 24 h. After drying at 100°C for 24 h, the carbon content of the support was determined by micro elemental analysis.

(c) *Polystyrene coating of support*

40 g of Norton Silica SS 5131 was refluxed for 1 hr in 100 ml toluene containing 4 g of (expandable) polystyrene. After cooling and superficial drying with tissue paper, this material was dried at 100°C for 24 h. The carbon content of this material (as determined by micro-elemental analysis) corresponds with 16 molecules of styrene $nm^{-2}$. An identical experiment with 16 g of expandable polystyrene in 100 ml of toluene resulted in a support containing 280 molecules of styrene $nm^{-2}$.

(d) The modified support particles prepared by the above procedures were then coated with *Pseudomonas aeruginosa* using the vacuum immobilisation technique as described in Example III(b), and the resulting immobilised systems assessed for their ability to oxidise methylcyclohexane to the corresponding alcohol as described in Example I. The results are set out in Table 8 below.

TABLE 8

| Support Coating $R_1$ | $R_2$ | $R_3$ | rate of product formation b) ($\mu$mole $h^{-1}$g dry cells$^{-1}$) | Lifetime (h) | Cell loading g dry cells. g support $^{-1}$ |
|---|---|---|---|---|---|
| | Uncoated | | 1.8 | > 60 | $7.4 \times 10^{-3}$ |
| $OC_2H_5$ | $(CH_2)_3NH_2$ | $OC_2H_5$ | 0.4 | < 40 | $8.9 \times 10^{-3}$ |
| $OC_2H_5$ | Phenyl | Phenyl | 1.8 | >140 | $3.6 \times 10^{-3}$ |
| $OC_2H_5$ | Phenyl | $OC_2H_5$ | 3.1 | >140 | $2.1 \times 10^{-3}$ |
| $OC_2H_5$ | $(CH_2)_7CH_3$ | $OC_2H_5$ | 4.3 | ≫120 | $1.0 \times 10^{-3}$ |
| $OC_2H_5$ | $(CH_2)_{11}CH_3$ | $OC_2H_5$ | 8.5 | ≫120 | $1.8 \times 10^{-3}$ |
| $OC_2H_5$ | $(CH_2)_{17}CH_3$ | $OC_2H_5$ | 6.5 | ≫120 | $2.2 \times 10^{-3}$ |
| Phenyl | $-CH=CH_2$ | Phenyl | 2.2 | >140 | $1.9 \times 10^{-3}$ |
| | Polystyrene coated | | 4.8 | ≫140 | $1.8 \times 10^{-3}$ |
| | Polystyrene coated | | 7.5 | ≫140 | $1.1 \times 10^{-3}$ |

**Claims**

1. A process for the microbial oxidation of a substantially water immiscible organic substrate, wherein a microorganism species capable of effecting the required oxidation of said substrate is located on a porous, water-bearing, particulate inert support, the pore size being greater than the mean diameter of the microorganism, thereby forming an immobilised system in which the microorganism has an aqueous environment, and the immobilised system is contacted with the substrate and a source of oxygen, characterised in that the source of oxygen is a gaseous mixture containing more than 21% but not more than 75% by vol. of oxygen.

2. Process as claimed in claim 1 wherein the substrate is an aliphatic hydrocarbon substrate.

3. Process as claimed in claim 2 wherein oxidation beyond the corresponding alkanol is inhibited by the addition of a different alcohol.

4. Process as claimed in claim 2 or 3 wherein the microorganism is *Pseudomonas aeruginosa*.

5. Process as claimed in claim 4 wherein the particle size of the inert support is at least 1 mm.

6. Process as claimed in any one of the preceding claims wherein the inert support is an inorganic oxide.

7. Process as claimed in any one of the preceding claims wherein the pore size of the inert support is greater than 0.5 micron.

8. Process as claimed in any one of the preceding claims wherein the support surface is provided with a lipophilic coating.

9. Process as claimed in any one of the preceding claims wherein the location of the microorganisms onto the support is effected by subjecting the support particles to reduced pressure, applying a paste of the microorganisms to the support whilst maintaining the reduced pressure, and subsequently restoring atmospheric pressure.

**Patentansprüche**

1. Verfahren zur mikrobiologischen Oxidation eines mit Wasser im wesentlichen nicht mischbaren organischen Substrats, bei dem eine Mikroorganismus-Art, die im Stande ist, die erforderliche Oxidation des Substrats durchzuführen, sich auf einem porösen, wasserhaltigen, teilchenförmigen inerten Träger befindet, dessen Porengröße größer ist als der mittlere Durchmesser des Mikroorganismus, wodurch ein immobilisiertes System entsteht, bei dem der Mikroorganismus in wäßriger Umgebung vorliegt, und das immobilisierte System mit dem Substrat und einer Sauerstoffquelle zusammengebracht wird, dadurch gekennzeichnet, daß die Sauerstoffquelle ein gasförmiges Gemisch ist, enthaltend mehr als 21 Vol.-%, aber nicht mehr als 75 Vol.-% Sauerstoff.

2. Verfahren nach Anspruch 1, wobei das Substrat ein aliphatisches Kohlenwasserstoffsubstrat ist.

3. Verfahren nach Anspruch 2, wobei die Oxidation über das entsprechende Alkanol hinaus durch Zusatz eines anderen Alkohols gehemmt wird.

4. Verfahren nach Anspruch 2 oder 3, wobei der Mikroorganismus Pseudomonas aeruginosa ist.

5. Verfahren nach Anspruch 4, wobei die Teilchengröße des inerten Trägers mindestens 1 mm beträgt.

6. Verfahren nach einem der vorangehenden Ansprüche, wobei der inerte Träger ein anorganisches Oxid ist.

7. Verfahren nach einem der vorangehenden Ansprüche, wobei die Porengröße des inerten Trägers mehr als 0,5 µm beträgt.

8. Verfahren nach einem der vorangehenden Ansprüche, wobei die Trägeroberfläche einen lipophilen Überzug besitzt.

9. Verfahren nach einem der vorangehenden Ansprüche, wobei die Ansiedelung der Mikroorganismen auf dem Träger durchgeführt wird, indem man die Trägerteilchen einem verminderten Druck aussetzt, eine Paste der Mikroorganismen auf den Träger aufbringt, während der verminderte Durck aufrechterhalten bleibt, und anschließend wieder Atmosphärendurck herstellt.

**Revendications**

1. Procédé d'oxydation microbienne d'un substrat organique essentiellement non miscible à l'eau, dans lequel une espèce de microorganisme capable d'effectuer l'oxydation nécessaire dudit substrat est située sur un support particulaire inerte, poreux, porteur d'eau, la porosité étant supérieure au diamètre moyen du micro-organisme, pour former ainsi un système immobilisé dans lequel le micro-organisme a un environnement aqueux, et le système immobilisé est mis au contact du substrat et d'une source d'oxygène, caractérisé en ce que la source d'oxygène est un mélange gazeux contenant plus de 21%, mais pas plus de 75%, en volume d'oxygène.

2. Procédé selon la revendication 1, dans lequel le substrat est un substrat hydrocarboné aliphatique.

3. Procédé selon la revendication 2, dans lequel l'oxydation au-delà de l'alcanol correspondant est inhibée par addition d'un alcool différent.

4. Procédé selon la revendication 2 ou 3, dans lequel le micro-organisme est *Pseudomonas aeruginosa*.

5. Procédé selon la revendication 4, dans lequel la granulométrie du support inerte est au moins égale à 1 mm.

6. Procédé selon l'une quelconque des revendications précédentes, dans lequel la support inerte est un oxyde minéral.

7. Procédé selon l'une quelconque des revendications précédentes, dans lequel la granulométrie du support inerte est supérieure à 0,5 micron.

8. Procédé selon l'une quelconque des revendications précédentes, dans lequel la surface du support est munie d'un revêtement lipophile.

9. Procédé selon l'une quelconque des revendications précédentes, dans lequel on effectue le positionnement des microorganismes sur le support en soumettant les particules de support à une pression réduite, en appliquant une pâte des micro-organismes sur le support tout en maintenant la pression réduite, puis en rétablissant la pression atmosphérique.